(19) **Europäisches Patentamt**
**European Patent Office**
**Office européen des brevets**

(11) Veröffentlichungsnummer: **0 270 001**
**A2**

(12) **EUROPÄISCHE PATENTANMELDUNG**

(21) Anmeldenummer: 87117469.4

(22) Anmeldetag: 26.11.87

(51) Int. Cl.⁴: **C07C 2/30**, C10M 107.10, C08F 110/14

(30) Priorität: **03.12.86 DE 3641237**

(43) Veröffentlichungstag der Anmeldung:
**08.06.88 Patentblatt 88/23**

(84) Benannte Vertragsstaaten:
**AT BE DE FR GB IT NL**

(71) Anmelder: **BASF Aktiengesellschaft**
**Carl-Bosch-Strasse 38**
**D-6700 Ludwigshafen(DE)**

(72) Erfinder: **Bronstert, Klaus, Dr.**
**Gartenstrasse 26**
**D-6719 Carlsberg(DE)**
Erfinder: **Mach, Helmut, Dr.**
**Dantestrasse 5**
**D-6900 Heidelberg(DE)**
Erfinder: **Rath, Hans Peter, Dr.**
**Friedhofstrasse 7**
**D-6718 Gruenstadt(DE)**
Erfinder: **Walter, Hans-Michael, Dr.**
**Im Haagweg 6**
**D-6701 Ruppertsberg(DE)**

(54) **Verfahren zur Herstellung von Decenoligomeren und deren Verwendung als Schmieröle.**

(57) Die Erfindung betrifft ein Verfahren zur Herstellung von Decenoligomeren durch Oligomerisieren von Decen-1 in Gegenwart eines Alkylaluminiumchloridkatalysators und eines Alkylhalogenidcokatalysators, wobei in einer ersten Reaktionsstufe das Decen-1 mit dem Katalysator unter Einhaltung einer Temperature zwischen 0 und +25°C gemischt und im gleichen Temperaturbereich mindestens 15 Minuten in Kontakt gehalten und in einer zweiten Reaktionsstufe der Cokatalysator bei einer Temperatur zwischen 0 und +35°C über einen Zeitraum von 15 bis 180 Minuten zugegeben wird sowie die Verwendung der Decenoligomeren, gegebenenfalls in hydrierter Form, als Grundflüssigkeit für synthetische Schmieröle.

EP 0 270 001 A2

## Verfahren zur Herstellung von Decenoligomeren und deren Verwendung als Schmieröle

Die Erfindung betriff ein Verfahren zur Herstellung von Decenoligomeren durch Oligomerisieren von Decen-1 in Gegenwart eines Alkylaluminiumchloridkatalysators und eines Alkylhalogenidcokatalysators bei niedrigen Temperaturen unter Ausschluß von Luft und Wasser und unter Rühren in einem Reaktor.

Bei derartigen Verfahren werden Decenoligomere erhalten, die für synthetische Schmieröle Verwendung finden.

Die bekannten Katalysatoren für die Herstellung der Decenoligomeren besitzen jedoch gewisse Nachteile, wie z.B. schlechte Löslichkeit, niedrige Umsätze des Decens, niedrige Oligomerisierungsgrade oder die Notwendigkeit, Katalysator und Cokatalysator in einem Lösungsmittel vorzumischen.

Aus den US-Patenten 3 637 503 und 3 725 498 ist bekannt, daß man Aluminiumhalogenidkatalysatoren in Kombination mit Cokatalysatoren (z.B. HCl, Carbonsäureester) einsetzt zur Oligomerisation von Olefinen. Diese Katalysatoren sind jedoch nur in beschränktem Maße löslich und beeinträchtigen die Reproduzierbarkeit. Auch im US-Patent 3 382 291 werden $BF_3$ /Cokatalysator-Kombinationen beschrieben, wobei für den Cokatalysator Wasser, Alkohol, Ether und andere organische Verbindungen in Frage kommen. Mit $BF_3$ werden jedoch vorrangig niedrige Oligomierisierungsgrade und damit niedrige Viskositäten der Reaktionsprodukte erreicht.

Im US-Patent 4 533 782 wird ein Verfahren beschrieben, bei dem eine Katalysator-Cokatalysator-Lösung, bestehend aus a) Aluminiumhalogenid oder aluminiumorganischer Verbindung, b) Alkylhalogenid und c) Lösungsmittel vorgemischt wird. Nachteilig bei diesem bekannten Verfahren sind das Vormischen der Katalysatorlösung und das Abtrennen von Lösungsmittel aus dem Reaktionsprodukt.

Aufgabe der vorliegenden Erfindung war es, die oben geschilderten Nachteile zu vermeiden und ein technisch einfaches Verfahrens zur möglichst vollständigen Oligomerisierung von Decen-1 aufzufinden.

Diese Aufgaben wurden durch Verfahren gemäß Patentansprüchen 1 bis 7 gelöst.

Die üblichen Verfahren zur Herstellung von Decenoligomeren mit Hilfe von Katalysator-/Cokatalysatorkombinationen der eingangs, beschriebenen Art sind, z.B. aus der oben zitierten Literatur, so bekannt, daß sich für den Fachmann eine weitere Erläuterung erübrigt. Als besonders geeignetes Alkylaluminiumhalogenid wird üblicherweise bevorzugt Ethylaluminiumdichlorid und als Alkylhalogenid vorzugsweise tert.-Butylchlorid verwendet. Unter Oligomeren werden bekanntlich Polymerisationsprodukte verstanden. die aus wenigen Monomeren bestehen, d.h. einen niedrigen Polymerisationsgrad aufweisen. Bevorzugt weisen die Decenoligomeren eine kinematische Viskosität zwischen 3 und 200 $mm^2/s$ bei 100°C, bestimmt nach DIN 51 562, auf.

Es wurde nun überraschend gefunden, daß unter Verwendung von Alkylaluminiumhalogenid als Katalysator und Alkylhalogenid als Cokatalysator durch zweistufiges Mischen von Decen-1 mit Alkylaluminiumhalogenid und anschließend mit Alkylhalogenid ein homogenes Reaktionssystem erhalten wird, in dem der Decen-1-Umsatz vollständig ist, so daß sich eine Rückführung von Decen-1 erübrigt.

Das erfindungsgemäße Verfahren zur Herstellung von Decenoligomeren durch Oligomerisierung von Decen-1 in Gegenwart eines Alkylaluminiumchloridcokatalysators unter Ausschluß von Luft und Wasser und unter Rühren in einem Reaktor wird so durchgeführt, daß in einer ersten Reaktionsstufe das Decen-1 mit dem Katalysator unter Einhaltung einer Temperatur zwischen 0 und +25, bevorzugt 5 und +15°C, gemischt und im gleichen Temperaturbereich mindestens 15, bevorzugt 30 bis 120 Minuten in Kontakt gehalten und in eine zweiten Reaktionsstufe der Cokatalysator bei einer Temperatur zwischen 0 und +35°C, bevorzugt +5 und 30°C, über einen Zeitraum von 15 bis 180. bevorzugt 15 bis 60 Minuten zugegeben wird.

Bei dem erfindungsgemäßen Verfahren wird der Cokatalysator portionsweise oder kontinuierlich zugegeben.

Die Reaktionswärme wird bei dem Oligomerisierungsverfahren durch Außenkühlung abegführt. so daß die Temperatur der Reaktionsmischung +35°C nicht übersteigt. Das Molverhältnis von Katalysator zu Cokatalysator liegt im Bereich von 1 : 0,5 bis 1 : 5,0, die Menge an Katalysator, bezogen auf Decen-1 im Bereich von 0,1 bis 5,0 Gewichtsprozent.

Die Alkylaluminiumhalogenide besitzen die allgemeine Formel $R_nAlX_{3-n}$, wobei R für Alkylrest, z.B. Methyl, Ethyl, Butyl usw. und X für Chlor oder Brom steht: n kann die Werte 1 oder 2 annehmen. Die Alkylhalogenide besitzen die allgemeine Formel R'X, wobei R' für einen Alkylrest, z.B. Ethyl. Propyl, n-Butyl, tert.-Butyl usw. und X für Chlor oder Brom steht. Bevorzugt ist eine Kombination von Ethylaluminiumdichlorid mit tert.-Butylchlorid.

Das Alkylaluminiumhalogenid kann gegebenenfalls in Decen-1 oder in Decenoligomeren gelöst eingesetzt werden.

Wie mit organometallischen Verbindungen üblich, müssen alle Einsatzstoffe und Apparaturen frei von Wasser und/oder Luft und anderen reaktiven Materialien sein. Hierzu kann das Decen-1 beispielsweise über ein Molekularsieb getrocknet und der Rührreaktor durch Ausheizen und Spülen mit trockenem Stickstoff inertisiert werden.

Die Aufarbeitung des Reaktionsansatzes erfolgt in bekannter Art und Weise dadurch, daß Katalysatorreste durch Waschen mit Wasser oder verdünnter Salzsäure entfernt werden.

Der Decenumsatz kann beispielsweise durch fraktionierte Destillation oder durch Gelpermeationschromatographie (GPC) bestimmt werden. Ein geeignetes GPC-System bestht aus 25 cm ®Lichrogel-Säule PS4 (Merck, Darmstadt), auf der das Reaktionsgemisch mit Tetrahydrofuran als Laufmittel mit einer Flußrate von einem ml/min getrennt wird. Die Detektion erfolgt z.B. mit einem Brechungsindexdetektor.

Bei synthetischen Schmierölen ist die Verwendung eines vollkommen gesättigten Materials erwünscht. Dazu kann das Reaktionsgemisch hydriert werden, um irgendwelche vorliegende Nichtsättigung zu vermeiden. Verfahren zur Herstellung synthetischer Schmiermitel auf Basis von Decenoligomeren sind in der US-Patentschrift 3 149 178 beschrieben.

Das folgende Beispiel dient lediglich zur Erläuterung der Erfindung, ohne sie zu begrenzen.

Beispiel

100 g Decen-1 (0,71 mol) wurden in einem 250 ml Vierhalskolben unter Stickstoffatmosphäre vorgelegt und auf +7°C gekühlt. Unter Rühren wurden 7,87 mmol $EtAlCl_2$ (1,11 Mol-% oder 1,00 Gew.% $EtAlCl_2$, bezogen auf Decen-1) mit Hilfe einer Glasspritze durch einen mit einer Gummikappe verschlossenen Hals des Reaktionskolbens zugegeben. Ohne Wärmetönung wurde 90 min bei +7 bis +8°C weitergerührt. Anschließend wurde innerhalb von 25 min 0,87 ml tert.-Butychlorid (0,73 g = 7,87 mmol) in 0,1 ml-Portionen zudoisert, wobei mit jeder Zugabe ein Temperaturanstieg beobachtet wurde (Temperaturmaximum 26°C). Danach wurde noch 65 min weitergerührt, die Temperature fiel dabei auf +7°C.

Es wurden Proben für die GPC nach 90, 120 und 180 min genommen. Danach ergibt sich folgende Zusammensetzung in Flächen-% (Fl.-%):

| Reaktionszeit (min) | Decen-1 (Fl.-%) | Oligomere (Fl.-%) |
|---|---|---|
| 90 | 35,31 | 64,69 |
| 120 | - | 100,00 |
| 180 | - | 100,00 |

Durch mehrmaliges Waschen mit Wasser und Trocknen über $MgSO_4$ wurde aufgearbeitet. Nach GPC und destillativer Bestimmung war der Decen-1-Umsatz quantitativ. Die kinematische Viskosität lag bei 20 $mm^2$/s bei 100°C, bestimmt nach DIN 51 562.

## Ansprüche

1. Verfahren zur Herstellung von Decenoligomeren durch Oligomerisieren von Decen-1 in Gegenwart eines Alkylaluminiumchloridkatalysators und eines Alkylhalogenidcokatalysators bei niedrigen Temperaturen unter Ausschluß von Luft und Wasser und unter Rühren in einem Reaktor, dadurch gekennzeichnet. daß in einer ersten Reaktionsstufe das Decen-1 mit dem Katalysator unter Einhaltung einer Temperatur zwischen 0 und +25°C gemischt und im gleichen Temperaturbereich mindestens 15 Minuten in Kontakt gehalten und in einer zweiten Reaktionsstufe der Cokatalysator bei einer Temperatur zwischen 0 und +35°C über einen Zeitraum von 15 bis 180 Minuten zugegeben wird.

2. Verfahren nach Anspruch 1, dadurch gekennzeichnet. daß die Temperatur in der ersten Reaktionsstufe zwischen 5 und +15°C gehalten wird.

3. Verfahren nach Anspruch 1, dadurch gekennzeichnet, daß das Decen-1 mit dem Katalysator 30 bis 120 Minuten in Kontakt gehalten wird.

4. Verfahren nach Anspruch 1, dadurch gekennzeichnet, daß der Cokatalysator portionsweise zugegeben wird.

5. Verfahren nach Anspruch 1, dadurch gekennzeichnet. daß der Cokatalysator kontinuierlich zugegeben wird.

6. Verfahren nach Anspruch 1, dadurch gekennzeichnet, daß die Temperatur in der zweiten Reaktionsstuffe zwischen 5 und 30°C gehalten wird.

7. Verfahren nach Anspruch 1, dadurch gekennzeichnet. daß der Zeitraum der Zugabe des Cokatalysators in der zweiten Reaktionsstufe zwischen 15 und 60 Minuten liegt.

8 .Verwendung der Decenoligomeren nach Anspruch 1, gegebenenfalls in hydrierter Form. als Grundflüssigkeit für synthetische Schmieröle.